Europäisches Patentamt

European Patent Office

Office européen des brevets

(19)

(11) Publication number: **0 249 432**
**A2**

# EUROPEAN PATENT APPLICATION

(12)

(21) Application number: **87305062.9**

(22) Date of filing: **09.06.87**

(51) Int. Cl.⁴: **C12N 15/00** , **A01H 1/00** , **A01G 7/00** , **C12N 5/00**

(30) Priority: **10.06.86 US 872532**

(43) Date of publication of application:
**16.12.87 Bulletin 87/51**

(84) Designated Contracting States:
**AT BE CH DE ES FR GB GR IT LI LU NL SE**

(71) Applicant: **CALGENE, INC.**
**1920 Fifth Street Suite F.**
**Davis California 95616(US)**

(72) Inventor: **Fillatti, Joanne J.**
**1225 Aspen Place**
**Davis California 95616(US)**
Inventor: **Thomas, Bruce R.**
**1302 Drexel Drive**
**Davis California 95616(US)**

(74) Representative: **Paget, Hugh Charles Edward et al**
**MEWBURN ELLIS & CO. 2/3 Cursitor Street**
**London EC4A 1BQ(GB)**

(54) Transformation and foreign gene expression with plant species.

(57) Plant species are produced by cocultivation transformation of cotyledon shoot cultures with a foreign gene followed by regeneration of plants from transformed cells, thereby producing plants capable of expressing the foreign gene. Particularly, tomato shoot cultures are employed and are transformed employing a manipulated Agrobacterium transformation system, followed by regeneration of the transformed plant tissue into plants.

EP 0 249 432 A2

## TRANSFORMATION AND FOREIGN GENE EXPRESSION WITH PLANT SPECIES

### BACKGROUND OF THE INVENTION

#### Field of the Invention

This invention relates to a method of transforming plant cells to introduce DNA, and provides a method for improving the genotypes and the phenotypes of plants. The method may be used for efficiently transforming plants to provide enhanced capabilities and/or novel phenotypes.

#### Background of the Invention

Breeding methods for plants have been limited due to the difficulty in moving genes between plant species. Therefore, the development of a method for genetic engineering with plant species is an attractive possibility. However, plant cells are substantially different from other types of cells in their requirements for a transforming system. First, unlike unicellular microorganisms, the plant cells have a low rate of proliferation. Second, the plant cells are much more sensitive to their environment in relation to viability, proliferation and regeneration to plants. Third, in order to determine whether the foreign gene has been functionally integrated into the plant cell, it is necessary to establish that the regenerated plant expresses the gene product. Finally, the plant cell has a strong rigid cell wall, making genetic engineering more difficult.

Because of the long time intervals involved between the manipulation of the plant cells and the demonstration of effective expression of the gene, it is essential that at each stage high efficiencies be achieved in transformation, cell growth, and regeneration. Furthermore, there are the additional considerations involved with matching a particular technique and the materials employed in that technique with the particular plant species. In addition, there is the further consideration of the subsequent handling of the transformed culture in order to obtain plants, namely plant regeneration from cells and callus.

#### Brief Description of the Relevant Literature

A.L. Gunay et al., Plant Science Letters (1978) 11:365-372, described in vitro regeneration from red pepper cotyledons. J.R. Liu et al., Plant Cell Organ Culture (1983) 2:293-304, described regeneration from apple seedling explants including cotyledons. B.R. Thomas etal., Theor. Appl. Genet. (1981) 59:215-219, describes the regeneration media for tomato. The 1985 Calgene U.S. Patent Application Serial No. 798,050 by Fillatti et al . describes the Agrobacterium of the present invention. The use of A. tumefaciens for transforming plants employing leaf disks is described in Horsch et al., Science (1985) 228 :1229-1231. See also, Herrera-Estrella et al ., Nature (1983 303:209-213; Fraley et al., Proc. Natl. Acad. Sci. USA (1983) 80:4803-4807; and Bevan et al ., Nature (1983) 304:184-187. The glyphosate resistant aroA gene is described in Stalker et al., J. Biol. Chem. (1985) 260 :4724-4728, while transcriptional initiation and termination regions are described by deGreve, J. Mol. Appl. Genet. (1983) 1 U:499-511; Salomon et al., EMBO J. (1984) 3:141-146; Velten et al ., ibid. (1984) 3:2723-2730; Garfinkel et al., Cell (1983) 27 :143-153; and Barker et al., Plant Mol. Bio. (1983) 2:335-350. Comai et al., Nature (1985) 317:741-744, describe the expression of a mutant aroA gene from Salmonella typhimurium in plants providing tolerance to glyphosate. Other reports discussing tomato cotyledon regeneration include: Orsay, French Theor. Appl. Genet. 68 (4), 1984, 317-322; Seeni, S. et al., Canadian J. Botany 59 (10), 1981, 1941-1943; and Vnuchkova, V.A., Fiziol Rast (Moscow, USSR), 24 (5), 1977, 1094-1100.

## SUMMARY OF THE INVENTION

Method for producing transformed plant species and resulting plant species containing novel nucleotide constructions capable of stable expression are provided. The methods are high efficiency transforming techniques using disarmed Agrobacterium and plant cotyledon tissue which is normally preincubated with medium conditioned by plant feeder cells. The method results in a high proportion of normal transformed cells, which are then efficiently regenerated into plants yielding both plants and seeds. The technique provides for stable expression of introduced genes, particularly foreign genes providing novel phenotypes for the plants.

## DESCRIPTION OF THE SPECIFIC EMBODIMENTS

Novel procedures and products are provided involving the introduction of novel nucleotide constructs into cells of plant species using plant cotyledon tissue. Regeneration of the transformed cells into plants, provides plants which express one or more genes present in the construct, so as to provide at least one property for the plant different from the original cultivar, particularly a phenotypic property.

The method employed uses injured cotyledon tissue, preincubated with conditioned medium. The preincubated plant cotyledonous tissue is cocultivated in an appropriate nutrient medium with disarmed transformed Agrobacterium having plant cell transformation capability and a DNA construct joined to a T-DNA border. The construct prepared by joining DNA fragments from diverse sources includes a gene capable of at least transcription in the host. The cocultivated cells are transferred to regeneration medium, normally having a bactericide selective for transformed cells, when a selectible marker is included in the construct. After shoots form, the shoots are transferred to a selective rooting medium to provide a complete plantlet. The plantlet may then be grown to provide seed, cuttings, or the like for propagating the novel plants. The method provides for high efficiency of transformation of plant cells and regeneration of plants from the cells.

The plant species which can be transformed are those plants employed for commercial purposes and subject to cultivation and management. Such plants include vegetables, fruits, ornamental flowers, woody species etc., such as the following di-, and polycotyledonous species: tomato, pinus, pepper, lettuce, cucumber, soybean, brassica (rapeseed), poplar, ornamental flowers, and the like.

Sterile seeds are employed as a source for plant cotyledons. The cotyledons may be mature or immature. The cotyledons may have protruded from the seed or be present in the seed and obtained by cutting the seed and disecting the cotyledons from the seed coat. For many plants, the cotyledons will have protruded, but for a few, e.g., pine, the cotyledons may be immature and obtained from within the seed.

Germination for protruding cotyledons will usually be at least four days in an appropriate germination medium and fewer than about fifteen days, preferably from about six to eight days. The resulting cotyledon tissue is used as a source of tissue for transformation, being injured to provide sites of regeneration. Regeneration occurs better at injured cell sites proximal to the basal site. Conveniently, the cotyledon may be cut into sections, usually three, and the middle section employed.

The injured cotyledon tissue is transferred to a feeder plate. The cells of the feeder plate act as a nurse culture for the injured cotyledon tissue and enhance the efficiency of transformation.

Any convenient plant cell suspension may be employed as the feeder culture, such as Nicotiana - (tobacco) or corn, particularly the former. While the tobacco feeder cells are preferred for certain crops, e.g., tomato, corn or other plant feeder cells are also useful as feeder cells. The preincubation with the feeder cell conditioned medium is usually at least six hours and not more than about 48 hours, 12 to 24 hours usually being employed.

The feeder plates are prepared by employing a plant suspension culture, e.g., Nicotiana cells, about $10^4$-$10^{10}$ cells/ml, usually $10^7$-$10^8$ cells/ml, in a soft agar medium, generally having from about 0.5 to 1% agar and containing an appropriate growth medium, such as Murashige and Skoog minimal organic medium and appropriate amounts of hormones, i.e., auxins, such as 2,4-dichlorophenoxyacetic acid (2,4-D), kinetin and vitamins, such as thiamine, with a medium appropriately buffered in the range from 5 to 6, preferably about 5.5. The kinetin and thiamine will generally be about 0.75 to 1.5mg/L, while the 2,4-D will generally be about 0.05 to 0.2mg/L. Desirably, the feeder plates are prepared prior to being used, usually at least about one, usually at least about two days, prior to being used.

The feeder plates (soft agar layers) are covered with a porous cover to prevent the feeder cells from coming into contact with the cotyledon explants. This porous cover allows the explants to be bathed in conditioned medium. This can be readily achieved employing a sterile filter paper disk. The cotyledons are then allowed to preincubate, followed by transfer to a broth culture of the Agrobacterium strain containing the DNA construct for transformation of the plant cells and the genetic capability for transfer of the construct into the plant cells. Generally, the number of bacteria are from about $10^6$ to $10^{10}$/ml, usually about $10^6$ to $10^8$ to $10^{10}$/ml and will vary with the particular strain.

The contact with the Agrobacterium in the bacterial broth culture, e.g., MG/L (same as LBMG; see Garfinkel et al., J. Bacteriol. (1980) 144:732-743), is usually at least about 1 minute and not more than about 1 hour, usually averaging about 30 minutes. The cotyledon sections are then transferred from the bacterial broth, excess surface liquid removed and the cotyledon sections returned to the feeder plates. Bacterial cocultivation on the feeder plates will usually be at least 6, usually at least 12 hours and not more than about four days, averaging about one to three days. After the cocultivation with the bacteria, the cotyledon segments are then transferred to regeneration media.

The regeneration media will usually contain a bactericide, e.g., carbenicillin (500mg/L), and may contain a selective reagent for selecting transformed cells. For example, with the kanamycin resistance gene (APH3'II), kanamycin will be added to at least about 30mg/L and usually not more than about 500mg/L, preferably from about 50 to 100mg/L, in the selective medium. The regeneration medium includes an appropriate salt source, such as Murashige-Skoog salts medium, a carbon source, e.g., sucrose, with appropriate other additives, such as hormones, e.g., zeatin, etc., at about 0.75-2.25mg/L, myo-inositol at about 50-200mg/L, etc. Also, a vitamin supplement may be added, e.g. Nitsch vitamins, at about 0.5 to 1.5ml/L of 1000x stock, (usually 1.0ml/L) as is conventional in regeneration media. The 1000x stock of Nitsch vitamins contains in a 100ml final volume: 50mg Thiamine HCl, 200mg glycine, 50mg Nicotinic Acid, 50mg Pyridoxine HCl, 50mg folic acid, 5ml Biotin and water to volume. The carbon source will be present in from 10 to 30g/L. Conveniently, the regeneration medium contains about 0.5 to 1.0% agar, with the regeneration medium being buffered at about pH 6 ± 0.5.

In 2 to 3 weeks shoots normally develop. When the shoots are approximately 1 to 2 cm, they are excised at the base and transferred to a rooting medium, which may be the same medium on which the seedlings were grown, with carbenicillin and kanamycin sulfate added.

Various disarmed strains may be employed which provide for efficient transformation of plants. the disarmed strains will lack in whole or in part the T-DNA region, particularly the hormone gene region, and may also lack one or both borders and the region associated with the expression of opines. Desirably, the Ti-or Ri-plasmid lacks a region of significant homology with the construct sequence.

The Agrobacterium system which is employed involves the use of a disarmed strain, for example, A. tumefaciens PC2760 (G. Coms et al., Plasmid (1982) 7:15-29; G. Coms et al., Gene (1981) 14:33; A. Hoekema et al., Nature (1983) 303:179-181; European Patent Application 84-200239.6, 2424183).

The Agrobacterium to be employed in the transforming of the plant cell will be transformed itself with a wide host range plasmid that can shuttle DNA from E. coli into Agrobacterium. This can be achieved by having a P-1 incompatibility plasmid replicon, e.g., RK2, and a plasmid replicon capable of providing multicopies in E. coli, usually at least 5, preferably at least 10, and up to 200 copies in E. coli. The wide host range plasmid will be characterized by having at least one T-DNA border sequence, particularly the right border sequence, or conveniently having both border sequences separated in one direction by the various constructs intended to be integrated into the plant species genome.

The transformed plant cells may be cells in culture, may be present as a disorganized mass in callus, organized as leaf explants, shoot cultures, seeds, fruits, leaves, roots, or organized as a whole plant. The foreign construct will normally be present in all or substantially all of the cells of the plant tissue, but expression may be limited to particular cells or particular stages in the development of the plant. The foreign construct will include transcriptional and translational initiation and termination signals, with the initiation signals 5' to the gene of interest and the termination signals 3' to the gene of interest.

The transcriptional initiation region which includes the RNA polymerase binding site (promoter) may be native to the plant host or may be derived from an alternative source, where the region is functional in the tomato host. Other sources include the Agrobacterium T-DNA genes, such as the transcriptional initiation regions for the biosynthesis of nopaline, octopine, mannopine, or other opine transcriptional initiation regions, transcriptional initiation regions from plants or other plant species than the host species, transcriptional initiation regions from viruses, particularly host specific viruses, or partially or wholly synthetic transcription initiation regions.

The transcriptional initiation regions may not only include the RNA polymerase binding site, but also regions providing for regulation of the transcription, where the regulation involves chemical or physical repression or induction, e.g., metabolites or light, or regulation based on cell differentiation, such as associated with leaves, roots, seed, or the like. Thus, the transcriptional initiation region or the regulatory portion of such region is obtained from an appropriate gene, which is regulated, for example, the 1,5-ribulosebiphosphatecarboxylase gene, which is light-induced, stress-induced genes, heat shock genes, which are temperature regulated, wound induced genes, meristem specific genes, etc.

The 3' termination region may be derived from the same gene as the transcriptional initiation region or a different gene. For example, where the gene of interest has a transcriptional termination region functional in the tomato species, that region may be retained with the gene.

An expression cassette is constructed which includes the transcriptional initiation region, the gene of interest under the transcriptional regulational control of the transcriptional initiation region, the initiation codon, the coding sequence of the gene, with or without introns, the translational stop codons, followed by the transcriptional termination region, which will include the terminator, and normally includes a polyadenylation signal sequence, and other sequences associated with transcriptional termination. The direction is 5'-3' in the direction of transcription. The cassette will usually be less than about 10kb, frequently less than about 6kb, usually being at least about 1kb, more usually being at least about 2kb.

The gene of interest may be derived from a chromosomal gene, cDNA, a synthetic gene, or combinations thereof. Where the expression product of the gene is to be located in other than the cytoplasm, the gene will usually be constructed to include particular amino acid sequences which result in translocation of the product to a particular site, which may be an organelle, such as the chloroplast, mitochondrion or nucleus, the cell plasma membrane, or may be secreted into the periplasmic space or into the external environment of the cell. Various secretory leaders, membrane integrator sequences, and translocation sequences for directing the peptide expression product to a particular site are described in the literature. See, for example, Cashmore et al., Biotechnology (1985) 3:803-808, Wickner and Lodish, Science (1985) 230:400-407.

Genes of interest for use in plant species include a wide variety of phenotypic and non-phenotypic properties. Among the phenotypic properties are enzymes which provide for resistance to stress, such as dehydration resulting from heat and salinity, resistance to insects, herbicides, toxic metal or trace elements, or the like. Resistance may be as a result of a change in the target site, enhancement of the amount of the target protein in the host cell, the increase in one or more enzymes involved with the biosynthetic pathway to a product which protects the host against the stress, and the like. Genes may be obtained from prokaryotes or eukaryotes, bacteria, fungi, e.g., yeast, viruses, plants, mammals or be synthesized in whole or in part. Illustrative genes include glyphosate resistant 3-enolpyruvylphosphoshikimate synthase gene, nitrilase, genes in the proline and glutamine biosynthetic pathway, metallothioneins, thioesterase II, acyl carrier protein, acetyl transacylase, etc. Other genes of interest may be involved with regulation of growth, such as manipulations of source/sink (carbon partitioning) relations, e.g., changes in solids content, or hormonal regulation, photosynthetic efficiency, such as altering the efficiency of RuBP carboxylase, or changing the quality of the plant taste or nutritional value, altering solid liquid ratios, viscosity or the number, size, color and abrasion resistance or firmness of the plant fruit.

One or more cassettes may be involved, where the cassettes may be employed in tandem for the expression of independent genes which may express products independently of each other or may be regulated concurrently, where the products may act independently or in conjunction.

The expression cassette to be transformed into plant cells by means of Agrobacterium , will be bordered usually within at least about 1kb by the right or both T-DNA borders. These borders may be obtained from any Ti-or Ri-plasmid and may be joined to the expression cassette by conventional ways. The expression cassette may be constructed so as to be directly transferred from a plasmid other than a Ti-or Ri-plasmid or may become integrated into the T-DNA region of a Ti-or Ri-plasmid through homologous recombination. Thus, the expression cassette could have DNA sequences at one or both borders of the expression cassette homologous with sequences present in the T-DNA region of the Ti-or Ri-plasmid. The Ti-plasmid will be disarmed so as to lack the genes expressing the protein product(s) essential to gall formation.

The expression cassette will normally be carried on a vector having at least one replication system. For convenience, it is common to have a replication system functional in E. coli, such as Col E1, pSC101, pACYC184, or the like. In this manner, at each stage after each manipulation, the resulting construct may be cloned, sequenced, and the correctness of the manipulation determined. In addition, or in place of the E. coli replication system, a broad host range replication system may be employed, such as the replication systems of the P-1 incompatibility plasmids, e.g., pRK290. These plasmids are particularly effective with disarmed Ti-plasmids for transfer of T-DNA to the plant species host.

In addition to the replication system, there will frequently be at least one marker present, which may be useful in one or more hosts, or different markers for individual hosts. That is, one marker may be employed for selection in a prokaryotic host, while another marker may be employed for selection in a eukaryotic host, particularly the plant species host. The markers may be protection against a biocide, such as antibiotics, toxins, heavy metals, or the like; or complementation, imparting prototrophy to an auxotrophic host. Various genes which may be employed include neomycin phosphotransferase (NPTII), hygromycin phosphotransferase (HPT), chloramphenicol aminotransferase (CAT), nitrilase, gentamicin resistance gene, etc. For plant host selection, markers of particular interest include NPTII, providing kanamycin resistance or G418 resistance, HPT, providing hygromycin resistance, CAT, providing chloramphenicol resistance, mutated aro A gene providing glyphosate resistance, etc.

The various fragments comprising the various constructs, expression cassettes, markers, and the like may be introduced consecutively by restriction enzyme cleavage of an appropriate replication system, and insertion of the particular construct or fragment into the available site. After ligation and cloning the vector may be isolated for further manipulation. All of these techniques are amply exemplified in the literature and find particular exemplification in Maniatis et al., Molecular Cloning: A Laboratory Manual, Cold Spring Harbor Laboratory, Cold Spring Harbor, NY, 1982.

The transformed plant cells containing the desired construct may now be isolated by appropriate selective means. The cells may be grown onto callus in a selective medium, which medium may contain a biocide, e.g., an antibiotic such as G418, hygromycin, bleomycin, etc., depending upon the particular marker included in the construct to provide for resistance. The concentration of the biocide will vary in accordance with plant cell susceptability. Where no marker is used or the expression of the marker gene proves inadequate for selection, transformed cells may be detected by Southern, Northern, or Western blots for detecting nucleic acid sequences and proteins.

Once the callus forms shoots, the shoots may be transferred to a rooting medium to produce plantlets which express the gene of interest.

The resulting plant may have a wide variety of desirable phenotypes, such as resistance to adverse conditions, e.g., heat, salinity, herbicides, etc, improved processing characteristics, improved organoleptic properties, overproduction of particular plant products, e.g. plant oils, production of bacterial or mammalian proteins and the like.

The following examples are offered by way of illustration and not by way of limitation.

## EXPERIMENTAL

E. coli strain MM294 (Hanahan, J. Mol. Biol. (1983) 116:557-580) was used as the host for binary vectors containing the pRK290 type replicon. Agrobacterium strain C58 has been described supra. PC2760 is another designation for Agrobacterium strain LBA4404 (Hoekema et al ., Nature (1983) 303:179-180). Strain K12 was generated by transforming pTiA6 into strain A114 (NT1) (Nester and Kosuge, Ann. Rev. Microbiol., (1981) 35:531, Hoekema et al., Nature (1983) 303 :179). Levels of antibiotics used with E. coli in mg/l were 30 for kanamycin, 50 for chloramphenicol, 300 for penicillin, 10 for tetracycline and 20 for gentamicin. Unless otherwise indicated, levels of antibiotics used with Agrobacterium in mg/l were 100 for kanamycin or gentamicin and 50 for carbenicillin or chloramphenicol.

## Laboratory Procedures

Restriction enzymes and T4 ligase were obtained from commercial sources and used according to manufacturers' recommendations. Standard methods of cloning and molecular analysis were performed as described in Maniatis et al., supra.

## EXAMPLE I

Plasmid Constructions

The BglII-SmaI fragment of Tn5 containing the entire structural gene for APHII (Jorgensen et al ., Mol. Gen. (1979) 177:65) was cloned into pUC8 (Vieira and Messing, Gene (1982) 19:259), converting the fragment into a HindIII-EcoRI fragment, since there is an EcoRI site immediately adjacent to the SmaI site. The PstI-EcoRI fragment containing the 3' portion of the APHII gene was then combined with an EcoRI-BamHI-SalI-Pst I linker into the EcoRI site of pUC7 (pCGN546W). Since this construct does not confer kanamycin resistance, kanamycin resistance was obtained by inserting the BglII-PstI fragment of the APHII gene into the BamHI-PstI site (pCGN546X). This procedure reassembles the APHII gene, so that Eco RI sites flank the gene. An ATG codon was upstream from and out of reading frame with the ATG initiation codon of APHII. The undesired ATG was avoided by inserting a Sau3A-PstI fragment from the 5'-end of APHII, which fragment lacks the superfluous ATG, into the BamHI-PstI site of pCGN546W to provide plasmid pCGN550.

The EcoRI fragment containing the APHII gene (1ATG) was then cloned into the unique EcoRI site of pCGN451, which contains an octopine synthase cassette for expression to provide pCGN552(1ATG).

Plasmid pCGN451 includes an octopine cassette which contains about 1556bp of the 5' non-coding region fused via an EcoRI linker to the 3' non-coding region of the octopine synthase gene of pTiA6. The pTi coordinates are 11,207 to 12,823 for the 3' region and 13,643 to 15,208 for the 5' region as defined by Barker et al., Plant Mol. Biol. (1983) 2 :325.

The 5' fragment was obtained as follows. A small subcloned fragment containing the 5' end of the coding region, as a BamHI-EcoRI fragment was cloned in pBR322 as plasmid pCGN407. The BamHI-EcoRI fragment has an XmnI site in the coding region, while pBR322 has two Xmn I sites. pCGN407 was digested with XmnI, resected with Bal31 nuclease and EcoRI linkers added to the fragments. After EcoRI and BamHI digestion, the fragments were size fractionated, the fractions cloned and sequenced. In one case, the entire coding region and 10bp of the 5' non-translated sequences had been removed leaving the 5' non-transcribed region, the mRNA cap site and 16bp of the 5' non-translated region (to a BamHI site) intact. This small fragment was obtained by size fractionation on a 7% acrylamide gel and fragments approximately 130 bp long eluted. This size fractionated DNA was ligated into M13mp9 and several clones sequenced and the sequence compared to the known sequence of the octopine synthase gene. The M13 construct was designated pI4, which plasmid was digested with BamHI and EcoRI to provide the small fragment which was ligated to a XhoI to BamHI fragment containing upstream 5' sequences from pTiA6 (Garfinkel and Nester, J. Bacteriol. (1980) 144:732) and to an EcoRI to Xho I fragment was cloned into the XhoI site of a pUC8 derivative, designated pCGN426. This plasmid differs from pUC8 by having the sole EcoRI site filled in with DNA polymerase I, and having lost the PstI and HindIII site by nuclease contamination of HincII restriction endonuclease, when a XhoI linker was inserted into the unique Hin cII site of pUC8. The resulting plasmid pCGN451 has a single EcoRI site for the insertion of protein coding sequences between the 5' non-coding region (which contains 1,550bp of 5' non-transcribed sequence including the right border of the T-DNA, the mRNA cap site and 16bp of 5' non-translated sequence) and the 3' region (which contains 267bp of the coding region, the stop codon, 196bp of 3' non-translated DNA, the polyA site and 1,153bp of 3' non-transcribed sequence).

The resulting plasmid pCGN451 having the ocs 5' and the ocs 3' in the proper orientation was digested with EcoRI and the Eco RI fragment from pCGN551 containing the intact kanamycin resistance gene inserted into the EcoRI site to provide pCGN552 having the kanamycin resistance gene in the proper orientation.

This ocs/KAN gene was used to provide a selectable marker for the trans type binary vector pCGN587.

The 5' portion of the engineered octopine synthase promoter cassette consists of TiA6 DNA from the XhoI fragment at bp 15208-13644 (Barker's numbering), which also contains the T-DNA boundary sequence (border) implicated in T-DNA transfer. In the plasmid pCGN587, the ocs/KAN gene from pCGN552 provides a selectable marker as well as the right border. The left boundary region was recloned from the HindIII-EcoI fragment as a KpnI-EcoRI fragment in pCGN565 to provide pCGN580. pCGN565 is a cloning vector based on pUC8-Cm, but containing pUC18 linkers. pCGN580 was linearized with BamHI and used to replace the smaller BglII fragment of pVCK102 (Knauf and Nester, Plasmid (1982) 8:45), creating pCGN585.

By replacing the smaller SalI fragment of pCGN585 with the XhoI fragment from pCGN552 containing the ocs/KAN gene, pCGN587 was obtained.

Construction of pPMG85

To construct pPMG85, the mannopine synthase gene (mas) 5′ region from pTiA6 (Salomon et al., EMBO J. (1984) 3:141-146). The gene was obtained from a cosmid clone carrying the T-DNAs of pTiA6 called pVCK232 (Knauf and Nester, Plasmid (1982) 8:45-54). pVCK232 was digested with EcoRI and one of the resulting fragments called Eco13 or EcoC was cloned in pACYC184 to provide plasmid pCGN14. Digestion of pCGN14 with ClaI and SphI yielded a mixture of fragments with the desired fragment resulting from cleavage at the ClaI site 20128 to the SphI site 21562 (Barker et al., Plant Mol. Bio. (1983) 2:335-350). This fragment contains the mas 5′ region and was cloned in pUC19 (Yanisch-Perron et al., Gene (1985) 33:103-119) which had been linearized with Sph I and AccI to provide plasmid pCGN40. The aro A BamHI fragment from pPMG34 (Stalker et al., J. Biol. Chem. (1985) 260:4724-4728) was cloned in the proper orientation in pCGN40, where the aroA gene was fused to the mas promoter region, providing pPMG67.

To provide a polyadenylation signal, the tml 3′ region of pTiA6 (Garfinkel et al ., Cell (1983) 27:143-153) was used. A T-DNA BamHI fragment (9062-13774; Barker numbering) containing such region was cloned from pVCK232 in pACYC184 in the orientation where nucleotide 13774 was proximal to the HindIII site of the vector. The resulting plasmid was digested with SmaI, which cleaves at nucleotide 11210 (Barker numbering) of the tml 3′ region and an octomeric XhoI linker (New England Biolabs) inserted. The resulting plasmid pBamX was digested with HindIII and XhoI and a fragment containing most of the mas 5′ region and the aroA gene obtained by digestion of pPMG67 with HindIII and SalI cloned into the linearized pBamX. The resulting plasmid pPMG73 contained 5′-mas -aroA-tml-3′ hybrid gene.

To allow for efficient selection in Agrobacterium, the kanamycin resistance gene from pUC4K (Vieira and Messing, Gene (1982) 19:259-268) was excised from SalI and cloned in a XhoI site present in the aroA distal end of the mas 5′ region giving pPMG76.

A 2.0kb EcoRI fragment in the Hind17 region of pRiA4T-LT-DNA (White and Nester, J. Bacteriol. - (1980) 141:1134; Taylor et al., Mol. Gen. Genet. (1985) 201:546) was cloned in the chloramphenicol resistance gene EcoRI site of pPMG76 yielding pPMG82.

To allow selection of transformed plants on kanamycin, a mas -npt hybrid gene was constructed. (See Velten et al., EMBO J. (1984) 3:2723-2730 for an analogous construction.) The mas 5′ region was excised from pCGN40 by digestion with Eco RV (21552; Barker numbering) and EcoRI (in the pUC19 polylinker) and cloned in pCGN451 digested with SmaI and EcoRI. The restriction deletes all of the ocs 5′ region from pCGN451 and inserts the mas 5′ region in its place. In addition, part of the pUC19 polylinker from XbaI to EcoRI is placed between the mas promoter region and the ocs polyadenylation site, allowing a choice of different sites for insertion of genes to be expressed. In the EcoRI site of this plasmid pCGN46, an EcoRI fragment of pCGN552, carrying the Tn5 npt gene (Rothstein et al., Cell (1980) 19:795-805), is inserted where an untranslated ATG sequence in the 5′ region had been removed.

The hybrid mas-npt-ocs gene was excised by digestion with XhoI and cloned in the Sal I site of pPMG82 resulting in pPMG85.

The resulting plasmid pPMG85 contained beginning from the EcoRI site, an EcoRI-HindIII 1.5kb fragment from pACYC184 and going clockwise, the bacterial kanamycin resistance gene from pUC4K, the mas 5′ region nucleotides 21476 to 20128, oriented in the clockwise direction, a BamHI-SalI aroA containing fragment from pPMG34, the tml 3′ region from nucleotides 11207 to 9062, a BamHI-SalI fragment from the tetracycline resistance gene of pACYC184, the npt gene from pCGN552, a 2.5kb SalI-Eco RI fragment from pACYC184 and a 2kb EcoRI fragment from pRIA4 on the BglII-HindIII-17 fragment (Huffman et al., J. Bacteriol. (1984) 157:269-276).

EXAMPLE II

Transformation of Tomato Cotyledon Tissue

Substantially sterile tomato cotyledon tissue was obtained from seedlings which had been grown at 24°C, with a 16hr/8hr day/night cycle in 100 x 25mm petri dishes containing Murashige-Skoog salt medium and 0.8% agar (pH 6.0). Any tomato species may be used, however, here the inbred breeding line was UC82B, available from the Department of Vegetable Crops, University of California, Davis, CA 95616. The cotyledons were cut into three sections and the middle placed onto feeder plates for a 24-hour preincubation. The feeder plates were prepared by pipetting 0.5ml of a tobacco suspension culture (~10⁶ cells/ml) onto 0.8% agar medium, containing Murashige minimal organic medium (K.C. Biologicals), 2,4-D (0.1mg/L), kinetin (1mg/L), thiamine (0.9mg/L) and potassium acid phosphate (200mg/L, pH 5.5). The feeder plates were prepared two days prior to use. A sterile 3mm filter paper disk containing feeder medium was placed on top of the tobacco cells after the suspension cells had grown for two days.

Following the preincubation period, the middle one third of the cotyledon sections were placed into a liquid MG/L broth culture (1-5ml) of the Agrobacterium tumefaciens strain 2760/587/85 (1x10$^7$-1x10$^8$ bacteria/ml). (The plasmids pCGN587 and pPMG85 were transformed into E. coli C2110 ( polA) and cointegrates selected by kanamycin and glyphosate resistance, de Fromard et al., BioTechnology, May 1983, pp. 262-267.) Bacterial matings were performed using two E. coli strains and one Agrobacterium strain. One E. coli strain (MM294) harbored pRK2073 which provided mobilization functions and the other strain (C2110) carried the plasmid with a kanamycin resistance marker to be transferred into Agrobacterium. The two E. coli strains were grown overnight at 37°C with shaking in LB broth. The Agrobacterium strain was grown overnight at 28°C in MG/L broth. Fifty microliters of each of the three strains were mixed on a nitrocellulose filter placed on an MG/L plate. The plate was incubated at 28°C for 3 days. The mixture was then streaked onto an AB minimal medium supplemented with 100µg/ml kanamycin and 100µg/ml streptomycin and incubated at 28°C for two days. Streptomycin was included to kill the two E. coli strains. Single colonies were picked and purified by two more streakings on the above medium. The cotyledon sections were cocultivated with the bacteria for 48hrs on the feeder plates and then transferred to regeneration medium containing 500mg/L carbenicillin and 100mg/L kanamycin. The regeneration medium is a K.C. Biologicals Murashige-Skoog salts medium with zeatin (2mg/L) myo-inositol (100mg/L), sucrose (20g/L), Nitsch vitamins and containing 0.8% agar (pH 6.0). In 2 to 3 weeks, shoots were observed to develop. When the shoots were approximately 1.25cm, they were excised and transferred to a Murashige and Skoog medium containing carbenicillin (500mg/L) and kanamycin (50mg/L) for rooting. Roots developed within 10-12 days.

Shoots which developed and subsequently rooted on media containing the kanamycin were tested for APH enzyme activity and for the presence of the aroA protein. Extracts of tomato plants showed a positive band for aroA protein in a Western blot. (Comai et al., Nature (1985) 37:341-344). Antibodies employed in the Western blot had been obtained by conventional procedures, immunizing a rabbit with the mutated glyphosate resistant aroA gene expression product. See U.S. Patent No. 4,535,060, which relevant disclosure is incorporated herein by reference. The presence of the correct band in the gel demonstrates that the glyphosate-resistant enzyme is produced and is stable in the host cell.

An aminoglycoside phosphotransferase enzyme (APH 3'II) assay was conducted on putative transformed tomato plants and shoots. Approximately 90% of the tomato plants which developed shoots and subsequently rooted on kanamycin containing medium tested positive for APH 3'II enzyme activity. APH 3'II confers resistance to kanamycin and neomycin. APH 3'II activity was assayed (Reiss et al., Gene (1984) 30 :211-218) employing electrophoretic separation of the enzyme from other interfering proteins and detection of its enzymatic activity by in situ phosphorylation of kanamycin. Both kanamycin and [γ-$^{32}$P] ATP act as substrates and are embedded in an agarose gel which is placed on top of the polyacrylamide gel containing the proteins. After the enzymatic reaction, the phosphorylated kanamycin is transferred to P-81 phosphocellulose ion exchange paper and the radiolabeled kanamycin is finally visualized by autoradiography. The Reiss et al. method was modified in the final washing of the P-81 ion exchange paper by rinsing in 0.1mg/ml of proteinase K.

Approximately 90-100% of the cotyledons plated onto "2Z" regeneration medium regenerated shoots after bacterial cocultivation of cotyledons. Approximately 60-90% of the cotyledons plated onto "2Z" medium with carbenicillin and kanamycin produced shoots. Approximately 80% of the shoots which initiated on the selective medium subsequently rooted on rooting mediumc containing kanamycin (50mg/ml). Over 90% of these shoots which rooted on kanamycin produced the aroA protein by western blot analysis of protein. Table 1 illustrates the transformation results for tomatoes.

TABLE 1

TOMATO TRANSFORMATION BY
COCULTIVATION WITH AGROBACTERIUM

| Treatment | No. of Cotyledons | | Total | |
|---|---|---|---|---|
| No Feeder | 13/19 | 68% | 27/41 | 66% |
| KCMS+ media | 14/22 | 64% | | |
| | | | | |
| Corn Feeder | 10/14 | 71% | 24/37 | 64.3% |
| KCMS+ media | 6/11 | 55% | | |
| | 8/12 | 67% | | |
| | | | | |
| Tobacco Feeder | 12/12 | 100% | 33/37 | 89.3% |
| KCMS+ media | 10/12 | 83% | | |
| | 11/13 | 85% | | |
| | | | | |
| No Feeder | 4/18 | 22% | 11/35 | 31.5% |
| N6 media | 7/17 | 41% | | |
| | | | | |
| Corn Feeder | 6/22 | 27% | 11/32 | 29.0% |
| N6 media | 5/10 | 31% | | |
| | | | | |
| Control no. | | | | |

KCMS+ media is tobacco media, N6 media is corn media.

A transformation event was determined by visually examining green shoots initiating growth from cotyledons on 2Z medium containing 100mg/L kanamycin.

Therefore, the tomato transformation/regeneration system described above was found to be rapid and efficient. Over 85% of the co-cultivated explants subsequently developed shoots on kanamycin selective medium and expressed the aroA protein. Between 2 and 10 shoots developed per explant. After 6 weeks, 10, 20 or even more shoots developed per explant. Glyphosate spray experiments confirmed that the resulting tomato plants were resistant to 0.75 Lbs/Acre glyphosate.

The above results demonstrate that plant species can be transformed efficiently, whereby foreign genes may be integrated into the plant genome and expressed, providing novel phenotypic properties. By virtue of the high transformation efficiency, successful transformations can be achieved within reasonable time periods and without unduly repetitive procedures. As evidenced by the above disclosure, plant species are provided which can be protected from herbicides, so that more efficient growth and production of crops can be achieved.

**Claims**

1. A method for transforming plant cells to introduce DNA into said plant cells, wherein the DNA becomes capable of replication and transcription in said cells, said method comprising:
cocultivating cotyledon cells, which were incubated with plant cell conditioned medium, with transformed disarmed Agrobacterium cells containing a construct which includes a T-DNA border and a gene of interest capable of transcription in plant cells, which construct is capable of being transferred into plant cells and becoming integrated into the genome of said plant cells, whereby said construct becomes integrated into the genome of said plant cells to provide transformed plant cells;

freeing said transformed plant cells of bacteria while incubating said transformed plant cells in a regeneration medium to produce shoots; and

transferring said shoots to a rooting medium to produce plantlets.

2. A method according to Claim 1, wherein said cotyledon cells are obtained by germinating a sterile seed under substantially sterile conditions to produce a protuding cotyledon; and

cutting said cotyledon into at least two pieces free of the remainder of said seed and ·selecting the cotyledon piece closer to the seed.

3. A method according to Claim 1 or Claim 2, wherein said conditioned medium is prepared by growing about $10^4$ to $10^{10}$ cells/ml in a soft agar medium with hormones and vitamins for at least about one day prior to incubation of said cotyledon cells.

4. A method according to any one of Claims 1 to 3, wherein said plant cells are first contacted with said Agrobacterium in a bacterial broth, followed by transferring the Agrobacterium contacted cells to conditioned medium.

5. A method for transforming tomato plant cells to introduce DNA into said tomato plant cells, wherein the DNA becomes capable of replication and transcription in said cells, said method comprising:

cocultivating cotyledon cells, which were incubated with plant cell conditioned medium, with transformed disarmed Agrobacterium cells containing a construct which includes a T-DNA border and a gene of interest capable of transcription in tomato plant cells, which construct is capable of being transferred into tomato plant cells and becoming integrated into the genome of said cells, whereby said construct becomes integrated into the genome of said tomato plant cells to provide transformed tomato plant cells;

freeing said transformed plant cells of bacteria while incubating said transformed plant cells in a regeneration medium to produce shoots; and

transferring said shoots to a rooting medium to produce plantlets.

6. A method according to Claim 5, wherein said cotyledon cells are obtained by germinating a sterile seed under substantially sterile conditions to produce a protuding cotyledon; and

cutting said cotyledon into at least two pieces free of the remainder of said seed and selecting the cotyledon piece closer to the seed.

7. A tomato cell having a construct coding for a foreign glyphosate resistant 3-enolpyruvylphosphoshikimate synthase.

8. A tomato cell having a foreign glyphosate resistant 3-enolpyruvylphosphoshikimate synthase.

9. A tomato cell according to Claim 7 or Claim 8, wherein said tomato cell is Lycopersicon esculentum.

10. Tomato plant seedlings comprising tomato cells according to Claim 8.